# EUROPÄISCHE PATENTSCHRIFT

(11) **EP 1 391 196 B1**
(45) Veröffentlichungstag und Bekanntmachung des Hinweises auf die Patenterteilung: **14.05.2008**
(21) Anmeldenummer: 03014580.9
(22) Anmeldetag: 07.07.2003
(51) Int. Cl.: A61K 8/02, A61K 8/81, A61Q 5/06, A61Q 5/12

(54) **Haarpflegeprodukt**
Hair care product
Produit capillaire

(30) Priorität: 17.08.2002 DE 10237737
(43) Veröffentlichungstag der Anmeldung: 25.02.2004
(73) Patentinhaber: Beiersdorf AG, 20245 Hamburg (DE)
(72) Erfinder: Demitz, Michael, 22529 Hamburg (DE); Argembeaux, Horst, 21465 Wentorf (DE); Rohde, Olaf, 22880 Wedel (DE)

(56) Entgegenhaltungen:
- EP-A- 0 867 167
- WO-A-02/096381
- DE-A- 19 809 942
- DE-C- 19 701 417
- GB-A- 2 180 215

## Beschreibung

Die vorliegende Erfindung betrifft ein Haarpflegeprodukt.

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten

Das Haar besteht aus dem frei aus der Haut herausragenden Haarschaft, dem keratinsierten (toten) Teil, der das eigentlich sichtbare Haar darstellt, und der in der Haut steckenden Haarwurzel, dem lebenden Teil, in dem das sichtbare Haar ständig neu gebildet wird. Der Haarschaft seinerseits ist aus drei Schichten aufgebaut: einem zentralen Teil, dem sogenannten Haarmark (Medulla), welches allerdings beim Menschen zurückgebildet ist und oft gänzlich fehlt, ferner dem Mark (Cortex) und der äußeren, bis zu zehn Lagen starken Schuppenschicht (Cuticula), die das ganze Haar umhüllt.

Das menschliche Haar ist, sofern keine krankhaften Veränderungen vorliegen, in seinem frisch nachgewachsenen Zustand praktisch nicht zu verbessern. Der in der Nähe der Kopfhaut befindliche Teil eines Haares weist eine nahezu geschlossene Schuppenschicht auf. Die Schuppenschicht als Außenhülle des Haares, aber auch der inneres Bereich unterhalb der Cuticula sind jedoch besonderer Beanspruchung durch Umwelteinflüsse ausgesetzt. Durch den Einfluss des Sonnenlichts,mechanisch Belastungen (intensives Kämmen oder Bürsten) oder Haarbehandlungen (Haarfärbungen z.B. Blondierungen, Haarverformungen z.B. Dauerwellen) wird die Schuppenschicht geschädigt bzw. zerstört. Mit zunehmenden Abstand von der Kopfhaut führt diese Schädigung zu einem teilweisen oder gar vollständigen Verlust der Schuppenschicht und damit zu gespaltenen Haarspitzen und anderen Schadensbildern (z.B. leicht elektrostatisch aufladbares Haar)

Das Ziel der Haarpflege ist es daher, Kopfhaut und -haar zu schützen und den Naturzustand des frisch nachgewachsenen Haares über einen möglichst langen Zeitraum zu erhalten oder, im Fall eines Verlusts, wieder herzustellen. Seidiger Glanz, geringe Porosität und ein angenehmes, glattes Gefühl (guter ""Griff") gelten als Merkmale für natürliches, gesundes Haar.

Aus diesen Gründen werden seit geraumer Zeit teils Haarpflegekosmetika angeboten, welche dazu bestimmt sind, nach Einwirken aus dem Haar wieder ausgespült zu werden, teils solche, welche auf dem Haar verbleiben. Diese können so formuliert werden, dass sie nicht nur der Pflege und dem Schutz des einzelnen Haars dienen, ach das Erscheinungsbild der Haartracht insgesamt verbessern, beispielsweise dadurch, dass sie das Haar geschmeidig und somit frisierwillig werden lassen, ihm einen angenehmen Griff geben und es seidig glänzend machen. Solche Zubereitung werden gemeinhin auch als Haarkonditionierer (engl. Conditioner) oder Haarkuren bezeichnet.

Haarkuren enthalten als Hauptwirkstoffe sogenannte Haarkonditioniermittel. Haarkönditioniermittei sind Zusätze zu Haarpflegemitteln, welche die Kämmbarkeit des nassen und des getrockneten Haares verbessern, die statische Aufladung des Haares neutralisieren und zugleich das Aussehen, die Griffigkeit, die Fülle, den Glanz und insgesamt das Frisiervermögen des Haares begünstigen. Die Wirkung eines Haarkonditioniermittels ist weitgehend von dem Aufziehvermögen der Substanz auf die Haaroberfläche abhängig.

Zu denHaärkonditioniermitteln zählen insbesondere die quaternären Ammoniumverbindungen, verschiedene Protein-Hydroiysate, aber auch,verschiedene Fette oder fettähnliche Produkte, z..B. die Silikone und die Silikon-Derivate, Glykole und kationische Polymere.

Natürliches Haar hängt meist schlaff und ohne Volumen vom Kopf herab. Ein weiteres Ziel der Haarpflege ist es daher, dem Haar Fülle und Frisur zu geben. Zur temporären Verformung des Haares und Gestaltung (engl. Styling) vielseitiger Frisuren verwendet man Haarfestiger, meist in Form von Schaumfestigern oder Haarsprays.Beide haarkosmetischen Mittel sind in Ihrer Zusammensetzung ähnlich, unterscheiden sich aber in ihrer Aufgabe und Anwendung. Schaumfestiger (auch Haarschaümfestiger genannt) werden in der Regel zur Frisurgestaltung im feuchten Haar verteilt, Haarsprays zur Fixierung auf die (trockene) fertig gestylte Frisur gesprüht. Neben den wegen ihrer leichten und angenehmen Anwendbarkeit zunehmend beliebten Schaumhaarfestigern und flüssigen Haarfestigern werden auch Haarfestigergele angeboten.

Die beiden Grundbausteine von Haarfestigern sind die Filmbildner und das Lösungsmittel. Als Filmbildner werden meist Mischpolymerisate (Polymere) aus Vinylpyrrolidon und Vinylacetat oder neutralisierter Crotonsäure eingesetzt. Auch andere kationische Polymere (d.h. in der Regel Polymere mit quaterem Stickstoffatomen) sowie anionische Polymere (z.B. Polymere mit Carboxylat Gruppen) können als Filmbildner eingesetzt Werden. Filmbildner bleiben nach dem Verdunsten des Lösungsmittels (meist Wasser und/oder Ethanol) ats Film auf dem Haar und fixieren dessen Form. Haarschaumfestiger enthalten zusätzlich Treibmittel wie Propan, n-Butah und/oder Isobutan.

Je nach gewünschter Festigkeit der Frisur werden unterschiedlich stark festigende Filmbildner in unterschiedlichen Mengenanteilen eingesetzt. Man unterscheidet daher Haarfestiger mit schwacher, normaler und starker Festigung sowie Festiger für normales, trockenes und fettiges Haar [W. Umbach (Hrsg.): Kosmetik, Entwicklung, Herstellung und Anwendung kosmetischer Mittel, 2. Aufl., Thieme Verlag, Stuttgart, 1995].

Ein Nachteil am Stande der Technik besteht in dem Umstand, dass bislang die Möglichkeiten, Wirkstoffe in Haarkuren einzuformulieren, begrenzt sind. Beispielsweise lassen sich Elektrolyte, Antioxidantien, UV-Filter, Vitamine und Feuchthaltemittel (engl. Moisturizer) nur unbefriedigend in Haarkuren und anderen Haarpftegezubereitungen einarbeiten. So führen die in der Haarpflege häufig eingesetzten Polymere und Elektrolyte zu einer Entmischung emulsionsartiger Zubereitungen. Auch sind einige Wirkstoffe wie z.B. eine Reihe von Vitaminen in wässrigen Zubereitungen instabil und damit nicht lange haltbar. Die Folge derartiger Formulierungsprobleme sind daher Haarpflegeprodukte, deren antioxidative, UV-Licht schützende, etc. Wirkung mangelhaft ist. Besonders schwierig gestalten sich in diesem Zusammenhang die Kombinationen aus Haarkur-Zubereitungen und Haarfestigern.

Nun könnten solche Produkte in Form von Einzelkomponenten (z.B. als Baukastensystem) dem Verbraucher angeboten werden. Aus Gründen der Anwendungsfreunlichkeit, besteht jedoch ein großes Interesse daran, derartige Haarpflegeprodukte in Form von "Kombipräparaten" anzubieten, die aus einer Verpackung heraus angewendet werden können.

Es war daher die Aufgabe der vorliegenden Erfindung, die Nachteile des Standes der Technik zu beseitigen und anwendungsfreundliche, hoch wirksame Haarpflegeprodukte zu entwickeln.

Überraschend gelöst wird die Aufabe durch ein kosmetisches Haarpflegeprodukt enthaltend
a) eine kosmetische Haarpflegezubereitung A in einer Menge von 75 bis 25 Gewichts-% der Gesamtzubereitung dadurch gekennzeichnet, dass in der Zubereitung A eine oder mehrere Verbindungen aus der Gruppe der C₈-C₂₂ Alkylhydroxy-Verbindungen in einer Gesamtkonzentration von 0,1 bis 10 Gewichts-%, bezogen auf das Gesamtgewicht der Zubereitung A, als Fettalkohole eingesetzt werden und eine oder mehrere Verbindungen aus der Gruppe der Alkylamine, ethoxilierten Amine, quartären Salze und Alkylimidazoline in einer Gesamtkonzentration von 0,1 bis 8 Gewichts-%, bezogen auf das Gesamtgewicht der Zubereitung A, als quartäre Ammoniumverbindungen eingesetzt werden,
b) eine Zubereitung B, die Wirkstoffe zur Haarpflege und/oder Frisurgestaltung enthält, in einer Menge von 25 bis 75 Gewichts-% der Gesamtzubereitung, wobei in Zubereitung B ein oder mehrere Verbindungen aus der Gruppe der nichtionischen, kationischen, anionischen oder amphoteren Polymere als Wirkstoffe zur Haarpflege und/oder Frisurgestaltung in einer Gesamtkonzentration von 0,01 bis 10 Gewichts-%, bezogen auf das Gesamtgewicht der Zubereitung B, eingesetzt werden,
   wobei
   i) die beiden Zubereitungen A und B in getrennten Kammern eines gemeinsamen Verpackungsbehältnisses aufbewahrt und aus diesem heraus angewendet werden,
   ii) beide Zubereitungen dem Verpackungsbehältnis entweder durch eine gemeinsame Öffnung gleichzeitig entnommen werden und beide Zubereitungen entweder vor dem Austritt aus der Entnahmeöffnung vermischt oder in Form einer streifenförmigen Gesamtzubereitung aus der Entnahmeöffnung austreten, oder zwei getrennten Öffnungen entnommen werden.

Die erfindungsgemäßen Haarpflegeprodukte zeichnen sich durch eine hohe haarpflegerische Wirksamkeit aus. Dabei sind sie überaus einfach anzuwenden, da das mühsame hantieren mit mehreren Pflegeprodukten aus unterschiedlichen Verpackungsbehältnissen entfällt. Die Dosierung der Zubereitungen wird vereinfacht, die Abfallmenge an zu entsorgenden Verpackungen ist reduziert. Die Produkte werden preisgünstiger und umweltfreundlicher in der Herstellung bzw. Entsorgung.

Zwar kennt der Stand der Technik die EP-A-867167 sowie die DE-A-19809942, doch konnten diese Schriften nicht den Weg zur vorliegenden Erfindung weisen.

Es ist erfindungsgemäß vorteilhaft, wenn als Zubereitung A eine Haarkur, ein Haarwasser, eine Frisiercreme, eine Frisierlotion, ein Frisiergel (z.B. Haargel, Wet-Look-Gel, Glittergel), eine Pomade, eine Schaumkur oder eine Haarspülung eingesetzt werden. Erfindungsgemäß bevorzugt ist der Einsatz einer Haarkur.

Erfindungsgemäß vorteilhafte Haarpflegeprodukte sind ferner dadurch gekennzeichnet, dass in Zubereitung B ein oder mehrere Verbindungen aus der Gruppe der nichtionischen, kationischen, anionischen oder amphoteren Polymere als Wirkstoffe zur Haarpflege und/oder Frisurgestaltung in einer Gesamtkonzentration von 0,01 bis 10 Gewichts-%, bevorzugt in einer Konzentration von 0,05 bis 5 Gewichts-% und ganz bevorzugt in einer Konzentration von 0,1 bis 4 Gewichts-%, jeweils bezogen auf das Gesamtgewicht der Zubereitung B, enthalten sind

Die erfindungsgemäßen Wirkstoffe können sowohl einzeln als auch in einer Kombination aus mehreren Wirkstoffen in der erfindungsgemäßen Zubereitung B vorliegen.

Die erfindungsgemäße kosmetische Zubereitung B kann ebenso wie die Zubereitung A aus einer wässrigen Lösung, einer Lipidmischung oder aus einer Emulsion bestehend. Eine erfindungsgemäße Zubereitung A und/oder B, welche in Form einer Emulsion vorliegt, kann erfindungsgemäß in Form eine W/O- (Wasser in Öl-), W/S- (Wasser in Silikonöl-), O/W- (Öl in Wasser-) oder S/W- (Silikonöl in Wasser-) Emulsion vorliegen. Ferner können sie erfindungsgemäß vorteilhaft auch in sogenannte multiplen Emulsionen wie beispielsweise W/O/W- oder O/W/O-Emulsionen vorliegen. Solche Formulierungen können vorzugsweise auch eine Mikroemulsion (z.B. eine PIT-Emulsion), eine Feststoff-Emulsiönen, (d. h. eine Emulsion, welche durch Feststoffe stabilisiert ist, z. B. eine Pickering-Emulsion), eine sprühbare Emulsion oder eine Hydrodispersion sein.

Es ist erfindungsgemäß bevorzugt, wenn als Zubereitung B ein Haarfestiger eingesetzt wird. Erfindungsgemäß besonders bevorzugt wird als Zubereitung B ein gelförmiger Haarfestiger eingesetzt.

Die erfindungsgemäßen Zubereitungen enthalten vorteilhafter Weise einen oder mehrere Konditionierer. Erfindungsgemäß bevorzugte Konditionierer sind beispielsweise alle Verbindungen, welche im International Cosmetic, Ingredient Dictionary and, Handbook (Volume 4, Herausgeber: R. C. Pepe, J.A. Wenninger, G. N. McEwen, TheCosmetic, Toiletry, and Fragrance Association, 9. Auflage, 2002) unter Section 4 unter den Stichworten Hair Conditioning Agents, Humectants, Skin-Conditioning Agents, Skin-Conditioning Agents-Emollient, Skin-Conditioning Agents-Humactant, Skin-Conditioning Agents-Miscellaneous, . Skin-Conditioning Agents-Occlusive und Skin Protectans aufgeführt sind sowie alle in der EP 0934956 (S.11-13) unter water soluble conditioning agent und oil soluble conditioning agent aufgeführten Verbindungen. Ein Teil dieser Verbindungen wird unter den Bestandteilen der wässrigen Phase und der Ölphase namentlich aufgeführt. Weitere erfindungsgemäß vorteilhafte Konditionierer stellen beispielsweise die nach der internationalen Nomenklatur für kosmetische Inhaltsstoffe (INCI) als Polyquaternium benannten Verbindungen dar (insbesondere Polyquaternium-1 bis Polyquaternium-56).

Es ist erfindungsgemäß vorteilhaft in Zubereitung B eine oder mehrere Verbindungen aus der Gruppe nichtionischen, anionischen, kationischen und amphoterer Polymere als Filmbildner eingesetzt werden. Die erfindungsgemäß bevorzugten Filmbildner sind Dabei nichtionische und amphotere Polymere. Erfindungsgemäß besonders bevorzugt ist der Einsatz der folgenden Filmbildner: PVP/VA Copolymer, PVP/Dimethylaminoethylmethacrylate Copolymers Dabei steht PVP für Polyvinylpyrrolidon und VA für Vinylacetat.

Die erfindungsgemäßen Filmbildner werden dabei vorteilhaft in einer/Gesamtkonzentration von 0,1 bis 10 Gewichts-%, bevorzugt einer Gesamtkonzentration von 0,5 bis 5,0 Gewichts-% und ganz besonders bevorzugt in einer Gesamtkonzentration von 0,8 bis 4,0 Gewichts-%, jeweils bezogen auf das Gesamtgewicht der Zubereitung B, eingesetzt.

In der erfindungsgemäßen Zubereitung A können erfindungsgemäß vorteilhaft eine oder mehrere Verbindungen aus der Gruppe der C₈-C₂₂ Alkylhydroxy-Verbindungen als Fettalkohole eingesetzt werden. Dabei ist der Einsatz von C₁₂-C₁₈-Fettalkoholen erfindungsgemäß bevorzugt und der Einsatz von C₁₆-C₁₈-Fettalkoholen erfindungsgemäß besonders bevorzugt.

Die erfindungsgemäßen Fettalkohole der Zubereitung A werden vorteilhaft in einer Konzentration von 0,1 bis 10 Gewichts-%, bevorzugt in einer Konzentration von 1 bis 8 Gewichts-% und ganz besonders bevorzugt in einer Konzentration von 1,5 bis 6 Gewichts-%, jeweils bezogen auf das Gesamtgewicht der Zubereitung A in dieser eingesetzt.

In der erfindungsgemäßen Zubereitung A können erfindungsgemäß vorteilhaft eine oder mehrere Verbindungen aus der Gruppe der ganz-, teil- oder nicht neutralisierten, verzweigten und/oder unverzweigten, gesättigten und/öder ungesättigten Fettsäuren mit einer Kettenlänge von 10 bis 40 C-Atomen, polyethoxilierte Fettsäureester, gesättigte und/oder ungesättigte, verzweigte und/oder unverzweigte Fettalkohole mit einer Kettenlänge von 10 bis 40 C-Atomen als Emulgatoren eingesetzt werden. Dabei ist der Einsatz von ganz-, teil- oder nicht neutralisierten, verzweigten und/oder unverzweigten, gesättigten und/oder ungesättigten Fettsäuren mit einer Kettenlänge von 10 bis 40 C-Atomen und polyethoxilierte Fettsäureester erfindungsgemäß bevorzugt. Erfindungsgemäß besonders bevorzugt ist der Einsatz von polyethoxilierte Fettsäurenester als Emulgatoren.

Die erfindungsgemäßen Emulgatoren werden erfindungsgemäß vorteilhaft in einer Gesamtkonzentration von 0,1 bis 10 Gewichts-%, bevorzugt in einer Konzentration vor 1,0 bis 5 Gewichts-% und ganz besonders bevorzugt in einer Konzentration von 1,5 bis 3 Gewichts-%, jeweils bezogen auf das Gesamtgewicht der Zubereitung A eingesetzt

In der erfindungsgemäßen Zubereitung A werden erfindungsgemäß vorteilhaft eine oder mehrere Verbindungen aus der Gruppe der Alkylamine, ethoxilierten Amine, quartären Salze und Alkylimidazoline als kationische Ammoniumverbindungen eingesetzt. Dabei sind quartäre Salze und Alkylimidazoline erfindungsgemäß bevorzugt und quatäre Ammoniumsalze erfindungsgemäß besonders bevorzugt.

Der oder die erfindungsgemäßen quartären Ammoniumverbindungen werden erfindungsgemäß vorteilhaft in einer Gesamtkonzentration von 0,1 bis 8 Gewichts-% bevorzugt in einer Gesamtkonzentration von 0,5 bis 5 Gewichts-% und ganz besonders bevorzugt in einer Gesamtkonzentration von 1 bis 4 Gewichts-%, jeweils bezogen auf das Gesamtgewicht der Zubereitung A, eingesetzt.

Die erfindungsgemäßen Zubereitungen A und B können als wässrige Lösung, verdickte wässrige Lösung oder wässrige Phase einer Emulsion neben Wasser erfindungsgemäß auch andere Inhaltsstoffe enthalten, beispielsweise Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder - monoethylether und analoge Produkte, ferner Alkohole niedriger C-Zahl, z.B. Ethanol, Isopropanol,1,2-Propandiol und Glycerin.

Die Ölphase bzw. Ölphasen der erfindungsgemäßen Formulierungen wird/werden vorteilhaft gewählt aus der Gruppe der polaren Öle, beispielsweise aus der Gruppe der Lecithine und der. Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnussöl, Rapsöl, Mandelöl, Palmöl, Kokosöl, Rizinusöl, Weizenkeimöl,Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere insektenwachse sowie Beerenwachs, Sheabutter und/oder Lanolin(Wollwachs).

Weitere vorteilhafte, polare Ölkomponeten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomeh und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 Solche Esteröle können dann vorteilhaft gewählt werden aus der Gruppe Octylpalmitat, Octylcocoat, Octylisostearat, Octyldodeceylmyristät, . Octyldodekanol, Cetearylisononanoat, Isopropylmyristat, isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat,- Stearylheptanoat, Oleyloleät, Öleylerucat, Erucyloleat, Erucylerucat, Tridecylstearat, Tridecyltrimellitat, sowie synthetische, halbsynthetische und natürliche Gemische solcher Ester, wie z.B.Jojobaöl.

Ferner kann eine Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Caprid/Diglycerylsuccinat, Butylenglykol Dicaprylat/Dicaprat, Cocoglyceride (z. B. Myritol® 331 von Henkel), C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat,Tricaprylin, Dimethylisosorbid. Es ist insbesondere vorteilhaft, wenn die Ölphase der erfindungsgemäßenFormulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche) Hexadecylbenzoat und Butyloctylbenzoat und Gemische davon *(Hallstar AB*) und/oder Diethylhexylnaphthalat (*Corapan®TQ von Haarmann & Reimer*).

Auch beliebige Abmischungen solcher Öl- und Wachskomponenten sind vorteilhaft in Sinne der vorliegendenErfindung einzusetzen.

Ferner kann eine Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann eine Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwässerstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, werden auch als Polydimethylsiloxan bzw. Dimethicon (INCl) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCl: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCl: Amodimethicone) und Silikonwachse, z. B. Polysiloxan-Polyalkylen-Copolymere (INCl: Stearyl Dimethicone und Cetyl Dimethicone) und DialkoxydimethylPolysitoxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Die erfindungsgemäßen Zubereitungen können auch alle nach der Kosmetikverordnung zugelassenenwasserlöslichen und/oder öllöslichen UV-A-, UV-B- und/oder Breitbandfiltersubstanzen enthalten

Die Zusammensetzungen enthalten gemäß der Erfindung außer den vorgenannten Substanzen gegebenenfalls die in der Kosmetik üblichen Zusatzstoffe, beispielsweise Parfüm, Farbstoffe, antimikrobielle Stoffe, rückfettende Agentien, Komplexierungs- und Sequestrierungsagentien, Perlglanzagentien, weitere Pflanzenextrakte, Vitamine, Wirkstoffe, Kohservierungsmittel, Bakterizide, Repellentien, Seibstbräuner, Depigmentie*rungsmittei, Pigmente, die eine färbende Wirkung haben, weichmachende, anfeuchtende und/oder feuchthaltende Substanzen, oder andere übliche Bestandteile einer kosmetischen oder dermatologischen Formulierung wie Emulgatoren, Polymere, Schaumstabilisatoren und Elektrolyte

Die erfindungsgemäßen Zubereitungen können erfindungsgemäß vorteilhaft ein oder mehrere Konservierungsstoffe enthalten. Vorteilhafte Konservierungsstoffe im Sinne der vorliegenden Erfindung sind beispielsweise Formaldehydabspalter (wie z. B. DMDM Hydantoin, welches beispielsweise unter der Handelsbezeichnung Glydant ™ von der Fa. Lonza erhältlich ist), Iodopropylbutylcarbamate - (z.B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfasst das Konservierungssystem erfindungsgemäß ferner vorteilhaft auch Konservierungshelfer, wie beispielsweise Octoxyglycerin, Glycine Soja etc. Die nachfolgende Tabelle gibt einen Überblick über einige erfindungsgemäß vorteilhafte Konservierungsstoffe:

| | | | |
|---|---|---|---|
| E 200 | Sorbinsäure | E 227 | Calciumhydrogensulfit. |
| E 201 | Natriumsorbat | E 228 | Kaliumhydrogensulfit) |
| E 202 | Kaliumsorbat | E 230 | Biphenyl (Diphenyl) |
| E 203 | Calciumsorbat | E 231 | Orthophenylphenol |
| E 210 | Benzoesäure | E 232 | Natriumorthophenylphenolat |
| E 211 | Natriumbenzoat | E 233 | Thiabendazol |
| E212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Calciumformiat |
| E 216 | p-Hydroxybenzoesäure-n-propylester | E 239 | Hexamethylentetramin |
| E 217 | p-Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |
| E 2.21 | Natriumsulfit : | E 280 | Propionsäure |
| E 222 | Natriumyhdrogensulfit | E 281 | Natriumpropionat |
| E 223 | Natriumdisulfit | E 282 | Calciumpropionat |
| E 224 | Kaliumdisulfit | E 283 | Kaliumpropionat |
| E 226 | Calciumsulfit | E 290 | Kohlendioxid |

Ferner vorteilhaft sind in der Kosmetik gebräuchliche Konservierungsmittel oder Konservierungshilfsstoffe, wie Dibromdicyanobutan (2-Brom-2-brommethylglutarodinitril), Phenoxyethanol, 3-lod-2-propinylbutylcarbamat, 2-Brom-2-nitro-propan=1,3-diol, Imidazolidinylharnstoff, 5-Chlor-2-methyl-4-isothiazolin-3-on, 2-Chloracetamid, Benzalkoniumchlorid, Benzylalkohol.
Es ist dabei erfindungsgemäß besonders bevorzugt, wenn als Konservierungsstoffe Benzoesäure und/oder Salicylsäure und/oder deren Derivate oder Salze eingesetzt werden.

Erfindungsgemäß vorteilhaft sind ein oder mehrere Konservierungsstoffe in einer Konzentration von 2 Gewichts-% oder kleiner 2 Gewichts-%, bevorzugt 1,5 Gewichts-% oder kleiner 1,5 Gewichts-% und besonders bevorzugt 1 Gewichts-% oder kleiner 1 Gewichts-%, jeweils bezogen auf das Gesamtgewicht der Zubereitung A oder B in einer der beiden Teilzubereitungen oder in beiden Teilzubereitungen enthalten.

Vorteilhaft liegen eine oder beide Komponenten gemäß der Erfindung in Form von Gelen vor und enthalten einen oder mehrere Gelbildner bzw. Hydrokolloide.

Hydrokolloid" ist dabei die technologische Kurzbezeichnung für die an sich richtigere Bezeichnung "hydrophiles Kolloid".

Als erfindungsgemäß vorteilhäfte Hydrokolloide werden Agar-Agar, Carrageen, Tragant Gummi arabicum, Alginate, Pektine, Polyosen, Guar-Meht, Johannisbrotbaumkernmehl, Stärke, Dextrine, Gelatine, Casein, Celluloseether, Hydroxyethyl- und; -propyl-cellulosederivate, Polysaccharide, Polyacryl- und Polyrnethacryl-Verbindüngen, Vinylpolymere Polycarbönsäuren, Polyether, Polyimin, Polyamide, Polykieselsäüren, Tonmineralien, Zeolithe, Kieselsäuren eingesetzt.

Erfindungsgemäß bevorzugte Hydrokolloide sind beispielsweise Methylcellulosen, als welche die Methylether der Cellulose bezeichnet werden. Sie zeichnen sich durch die folgende Strukturformel aus 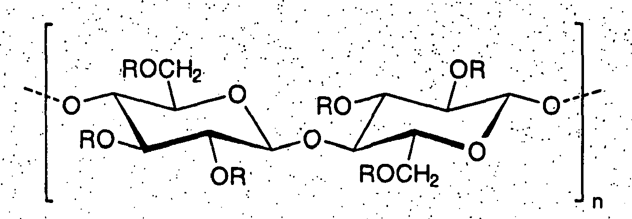 in der R ein Wasserstoff oder eine Methylgruppe darstellen kann.

Insbesondere vorteilhaft im Sinne der vorliegenden Erfindung sind die im allegemeinen ebenfalls als Methylcellulosen bezeichneten, Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, beispielsweise die unter der Handelsbezeichnung Methocel E4M bei der Dow Chemical Comp. erhältlichen.

Erfindungsgemäß ferner vorteilhaft ist Natriumcarboxymethylcellulose, das Natrium-Satz des Glykolsäureethers der Cellulose, für welches R in Strukturformel I ein Wasserstoff und/oder CH2-COONa darstellen kann. Besonders bevorzugt ist die unter der Handelsbezeichnung Natrosol Plus 330 CS bei Aqualon erhältliche, auch als Cellulose Gum bezeichnete Natriumcarboxymethylcellulose.

Bevorzugt im Sinne der vorliegenden Erfindung ist ferner Xanthan (CAS-Nr: 11138-66-2), auch Xanthan Gummi genannt, welches ein anionisches Heteropolysaccharid ist, das in der Regel durch Fermentation aus Maiszucker gebildet und als Kaliumsalz isoliert wird. Es wird von Xanthomonas campestris und einigen anderen Spezies unter aeroben Bedingungen mit einem Molekulargewicht von 2x106 bis 24x106 produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat .. und Pyruvat. Xanthan ist die Bezeichnung für das erste mikrobielle anionische Heteropolysaccharid. Es wird von Xanthomonas campestris und einigen anderen Spezies unter aeroben Bedingungen mit einem Molekulargewicht von 2-15 106 produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Die Anzahl der Pyruvat-Einheiten bestimmt die Viskosität des Xanthans. Xanthan wird in zweitägigen Batch-Kulturen mit einer Ausbeute von 70-90 %, bezogen auf eingesetztes Kohlenhydrat, produziert. Dabei werden Ausbeuten von 25-30 g/t erreicht. Die Aufarbeitung erfolgt nach Abtöten der Kultur durch Fällung mit z. B. 2-Propanol. Xanthan wird anschließend getrocknet und gemahlen.

Vorteilhafter Gelbildner im Sinne der vorliegenden Erfindung ist ferner Carrageen, ein gelbildender und ähnlich wie Agar aufgebauter Extrakt aus nordatlant., zu den Florideen zählenden Rotalgen (Chondrus crispus u. Gigartina stellata)

Häufig wird die Bezeichnung Carrageen für das getrocknete Algenprodukt und Carrageenan für den Extrakt aus diesem verwendet. Das aus dem Heißwasserextrakt der Algen ausgefällte Carrageen ist ein farbloses bis sandfarbenes Pulver mit einem Molekulargewichtsbereich von 100 000-800 000 und einem Sulfat-Gehalt von ca. 25 % Carrageen, das in warmem Wasser sehr leicht löslich ist; beim Abkühlen bildet sich ein thixotropes Gel, selbst wenn der Wassergehalt 95-98 % beträgt. Die Festigkeit des Gels wird durch die Doppelhelix-Struktur des Carrageens bewirkt . Beim Carrageenan unterscheidet man drei Hauptbestandteile: Die gelbildende κ-Fraktion besteht aus D-Galactose-4-sulfat und 3,6-Anhydro-α-D-galactose, die abwechselnd in 1,3- und 1,4-Stellung glykosidisch verbunden sind (Agar enthält demgegenüber 3,6-Anhydro-α-L-galactose). Die nicht gelierende λ-Fraktion ist aus 1,3-glykosidisch verknüpften D-Galactose-2-sulfat und 1,4-verbundenen D-Galactose-2,6-disUlfat-Resten zusammengesetzt u. in kaltem Wasser leicht löslich. Das aus D-Galactose-4-sulfat in 1,3-Bindung und 3,6-Anhydro-α-D-galactose-2-sulfat in 1,4-Bindung aufgebaute :1.-Carrageehan ist sowohl wasserlöslich als auch gelbildend. Weitere Carrageen-Typen werden ebenfalls zeit griechischen Buchstaben bezeichnet: α, β, γ, µ, ν, ξ, π, ω, χ. Auch die Art vorhandener Kation nen (K+, NH4+, Na+, Mg2+, Ca2+) beeinflusst die Löslichkeit der Carrageene.

Polyacrylate sind ebenfalls vorteilhaft im sinne der vorliegenden Erfindung zu verwendende Gelatoren. Erfindungsgemäß vorteilhafte Polyacrylate sind Acrylat-Alkylacrylat-Copolymere, insbesondere solche, die aus der Gruppe der sogenannten Carbomere oder Carbopole (Carbopol® ist eigentlich eine eingetragene Marke der NOVEON Inc.) gewählt werden. Insbesondere zeichnen sich das oder die erfindungsgemäß vorteilhaften Acrylat-Alkylacrylat-Copolymere durch die folgende Struktur aus:

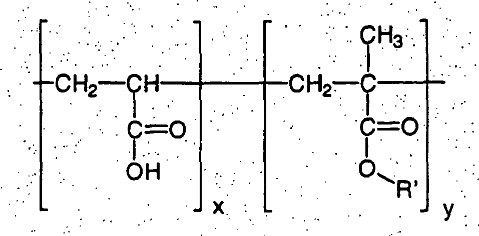

Darin stellen R' einen langkettigen Alkylrest und x und y Zahlen dar, welche den jeweiligen stöchiometrischen Anteil der jeweiligen Comonomere symbolisieren

Erfindungsgemäß besonders bevorzugt sind Acrylat-Copolymere und/oder Acrylat-Alkylacrylat-Copolymere, welche unter den Handelbezeichnungen Carbopol® 1382, Carbopol® 981 und Carbopol® 5984, Aqua SF-1 von der NOVEON Inc. bzw. als Aculyn® 33 von International Specialty Products Corp. erhältlich sind.

Ferner vorteilhaft sind Copolymere aus C10-30-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Vorteilhaft sind Verbindungen, die die INCI-Bezeichnung "Acrylates/C 10-30 Alkyl Acrylate Crosspolymer" tragen. Insbesondere vorteilhaft sind die unter den Handelsbezeichhungen Pemulen TR1 und Pemulen TR2 bei der NOVEON Inc. erhältlichen.

Vorteilhaft sind ferner Verbindungen, die die INCI-Bezeichnung "acrylates/C12-24 pareth-25 acrylate copolymer" (unter der Handelsbezeichnungen Synthalen® W2000 bei der 3V Inc. erhältlich), die die INCI-Bezeichnung "acrylates/steareth-20 methacrylate copolymer" (unter der Handelsbezeichnungen Aculyn® 22 bei der international Specialty Products Corp. erhältlich), die die INCI-Bezeichnung "acrylates/steareth-20 itaconate copolymer" (unter der Handelsbezeichnungen Structure 2001® bei der National Starch erhältlich), die die INCI-Bezeichnung "acrylates/aminoacrylates/C10-30 alkyl PEG-20 itaconate copolymer" (unter der Handelsbezeichnungen Structure Plus®, bei der National Starch erhältlich) und ähnliche Polymere.

Erfindungsgemäß bevorzugt ist es insbesondere, neutralisierte oder teilneutralisierte Polyacrylate (z.B. Carbopole der Firma Noveon) einzusetzen.

Es ist vorteilhaft im Sinn der vorliegenden Erfindung, wenn der Gehalt an einem mehreren Polyacrylaten in einer kosmetischen oder dermatologischen Zubereitung aus dem Bereich von 0,1 bis 8 Gew.-%, ganz besonders vorteilhaft von 0,1 bis 5 Gew.-% gewählt wird, jeweils bezogen auf das Gesamtgewicht der jeweiligen Zubereitung.

Erfindungsgemäß besonders bevorzugt werden als Hydrokolloid Xanthangummi, Gellangummi, Polyacrylate und/oder Polyacrylat-Copolymere eingesetzt.

Erfindungsgemäß vorteilhaft beträgt der pH-Wert der Zubereitung A von 2 bis 5, bevorzugt von 3 bis 4 und besonders bevorzugt 3,5. Erfindungsgemäß vorteilhaft beträgt der pH-Wert der Zubereitung B von 4 bis 8, bevorzugt von 5 bis 7 und besonders bevorzugt 6,5.
Der pH-Wert läßt sich dabei mit den dafür in der Kometik üblicherweise verwendete Säuren und Basen einstellen.

Erfindungsgemäße Verpackungsbehältnisse können vorteilhaft aus einer Kunststoffflasche oder einer Aerosoldose bestehen. Erfindungsgemäß vorteilhaft kann auch mindestens eine der beiden Zubereitungen mit einem Treibmittel, aufgeschäumt werden.

Als erfindungsgemäß vorteilhafte Verpackungsbehältnisse können beispielsweise Verpackungen gewählt werden, wie sie in der WO 96/37420 oder der GB 2 180 215 offenbart sind. Auch Verpackungssysteme wie sie von mehrfarbig gestreifter Zahnpasta her bekannt sind, lassen sich erfindungsgemäß vorteilhaft verwenden unter der Maßgane, dass die beiden Zubereitungen durch eine undurchlässige Sperre voneinander getrennt sind und erst beim Austritt aus der Verpackung zu der bekannten streifenförmigen Gesamtzusammensetzung zusammengesetzt werden.

Vorteilhafte Verpackungsbehältnisse stellen ferner mit zwei Kammern versehende Quetschflaschen dar, die entweder für beide Zubereitungen eine gemeinsame Entnahmeöffnung oder aber zwei separate Entnahmeöffnungen aufweisen.

Erfindungsgemäß vorteilhafte Verpackungsbehältnisse stellen auch Doppelkammertuben mit ein oder zwei Entnahmeöffnungen dar. Nicht zuletzt ist es erfindungsgemäß vorteilhaft, wenn beide Zubereitungen zusammen oder getrennt mit Hilfe eines Pump-und/oder Dosierspenders dem:Verpackungsbehältnis entnommen werden können.

Erfindungsgemäß vorteilhaft ist das Verpackungsbehältnis ganz oder teilweise aus transparentem Material, so dass die Schwebkörper und/oder Effektstoffe bereits in der Verpackung optisch wahrgenommen werden können.

Erfindungsgemäß vorteilhaft sind beide Kammern des Verpackungsbehältnisses nebeneinander oder ineinander angeordnet.

Generell bevorzugt sind Verpackungsbehältnisse, bei denen die beiden erfindungsgemäßen Zubereitungen A und B dem Behältnis aus zwei unterschiedlichen Entnahmeöffnungen entnommen werden.

Es ist erfindungsgemäß vorteilhaft, wenn die beiden Zubereitungen A und B unterschiedlich gefärbt und/oder mit unterschiedlichen Effektstoffen beladen sind.

Erfindungsgemäß ist die Verwendung des erfindungsgemäßen Haarpflegeproduktes zur Reinigung und Pflege der Haare und der Kopfhaut.

Insbesondere ist die Verwendung eines erfindungsgemäßen Haarpflegeproduktes als Frisurgestaltungsmittel ("Styling"-Mittel), Haarkur und/oder Konditioniermittel erfindungsgemäß vorteilhaft.

Da der erfindungsgemäße Einsatz des erfindungsgemäßen Haarpflegemittelszu keineswegs nur auf das Haupthaar des Menschen beschränkt ist, ist auch die Verwendung des erfindungsgemäßen Haarpflegemittels zur Pflege des Haarkleides von Säugetieren mit Haarkleid und/oder Fell erfindungsgemäß.

Ferner eignen sich die erfindungsgemäßen Produkte hervorragend zur Pflege von Gegenständen des täglichen Lebens (z.B. Kleidungsstücke, Wäsche, Textilien).

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispielrezepturen der erfindungsgemäßen -Haarpflegezubereitung A

| | **1** | **2** | **3** |
|---|---|---|---|
| Hydroxypropylmethylcellulose | 0,5 | 0,5 | 0,5 |
| Cetyltrimethylammohium Chlorid | 1,3 | 1,0 | 1,5 |
| Glycerin | 3,0 | 2,0 | 2,8 |
| Cetearylalkohol | 2,5 | 2,5 | 2,5 |
| Glycerylstearat | 2,0 | 2,0 | 2,0 |
| Polyquaterniüm-10 | 0,1 | - | - |
| Guar Hydroxypropy Trimonium Chlorid | - | 0,2 | - |
| Konservierung; Parfum, Puffer | q.s. | q. s. | q.s. |
| Wasser | ad 100 | ad 100 | ad 100 |

### Beispielrezepturen der erfindungsgemäßen Haarpflegezubefältung B

| | **1** | **2** |
|---|---|---|
| PVP/VA Copolymer | 2,0 | - |
| PVP/Dimethylaminoethylmethacrylat | - | 3,0 |
| Dimethicone Copolyol | 0,2 | 0,3 |
| Carbomer | 1,0 | 1,0 |
| Ethoxiliertes Rizinusölhydrolysat | 0,2 | 0,2 |
| Ethanol | - | 10 |
| Konservierung, Puffer, Parfum | q.s. | q.s. |
| Wasser | ad 100 | ad 100 |

## Patentansprüche

1. Kosmetisches Haarpflegeprodukt enthaltend
a) eine kosmetische Haarpflegezubereitung A in einer Menge von 75 bis 25 Gewichts-% der Gesamtzubereitung **dadurch gekennzeichnet, dass** in der Zubereitung A eine oder mehrere Verbindungen aus der Gruppe der C₈-C₂₂ Alkylhydroxy-Verbindungen in einer Gesamtkonzentration von 0,1 bis 10 Gewichts-%, bezogen auf das Gesamtgewicht der Zubereitung A, als Fettalkohole eingesetzt werden und eine oder mehrere Verbindungen aus der Gruppe der Alkylamine, ethoxilierten Amine, quartären Salze und Alkylimidazoline in einer Gesamtkonzentration von 0,1 bis 8 Gewichts%, bezogen auf das Gesamtgewicht der Zubereitung A, als quartäre Ammoniumverbindungen eingesetzt werden,
b) eine Zubereitung B, die Wirkstoffe zur Haarpflege und/oder Frisurgestaltung enthält, in einer Menge von 25 bis 75 Gewichts-% der Gesamtzubereitung, wobei in Zubereitung B ein oder mehrere Verbindungen aus der Gruppe der nichtionischen, kationischen, anionischen oder amphoteren Polymere als Wirkstoffe zur Haarpflege und/oder Frisurgestaltung in einer Gesamtkonzentration von 0,01 bis 10 Gewichts-%, bezogen auf das Gesamtgewicht der Zubereitung B, eingesetzt werden,
wobei
i) die beiden Zubereitungen A und B in getrennten Kammern eines gemeinsamen Verpackungsbehältnisses aufbewahrt und aus diesem heraus angewendet werden,
ii) beide Zubereitungen dem Verpackungsbehältnis entweder durch eine gemeinsame Öffnung gleichzeitig entnommen werden und beide Zubereitungen entweder vor dem Austritt aus der Entnahmeöffnung vermischt oder in Form einer streifenförmigen Gesamtzubereitung aus der Entnahmeöffnung austreten, oder zwei getrennten Öffnungen entnommen werden.

2. Haarpflegeprodukt nach Anspruch 1, **dadurch gekennzeichnet, dass** als Zubereitung A eine Haarkur eingesetzt wird.

3. Haarpflegeprodukt nach einem der Ansprüche 1 oder 2, **dadurch gekennzeichnet, dass** als Zubereitung B ein Haarfestiger eingesetzt wird.

4. Haarpflegeprodukt nach einem der Ansprüche 1 bis 3, **dadurch gekennzeichnet, dass** als Zubereitung B ein gelförmiger Haarfestiger eingesetzt wird.

5. Haarpflegeprodukt nach einem der Ansprüche 1 bis 4, **dadurch gekennzeichnet, dass** in der Zubereitung A eine oder mehrere Verbindungen aus der Gruppe der ganz-, teil- oder nicht neutralisierten, verzweigten und/oder unverzweigten, gesättigten und/oder ungesättigten Fettsäuren mit einer Kettenlänge von 10 bis 40 C-Atomen, polyethoxilierte Fettsäureester, gesättigte und/oder ungesättigte, verzweigte und/oder unverzweigte Fettalkohole mit einer Kettenlänge von 10 bis 40 C-Atomen in einer Gesamtkonzentration von 0,1 bis 10 Gewichts-%, bezogen auf das Gesamtgewicht der Zubereitung A, als Emulgatoren eingesetzt werden.

6. Haarpflegeprodukt nach einem der Ansprüche 1 bis 5, **dadurch gekennzeichnet, dass** in Zubereitung B als Wirkstoffe Filmbildner eingesetzt werden.

7. Haarpflegeprodukt nach einem der Ansprüche 1 bis 6, **dadurch gekennzeichnet, dass** als Polymere in der Zubereitung B ein oder mehrere Verbindungen aus der Gruppe der nichtionischen und amphoteren Polymere als Wirkstoffe zur Haarpflege und/oder Frisurgestaltung eingesetzt werden.

8. Haarpflegeprodukt nach einem der Ansprüche 1 bis 7, **dadurch gekennzeichnet, dass** als Polymere in der Zubereitung B ein oder mehrere Verbindungen aus der Gruppe PVPNA Copolymer, PVP/Dimethylamino-ethylmethacrylate Copolymer eingesetzt werden.

9. Haarpflegeprodukt nach einem der Ansprüche 1 bis 8, **dadurch gekennzeichnet, dass** als Verbindungen aus der Gruppe Alkylamine, ethoxilierten Amine, quartären Salze und Alkylimidazoline der in der Zubereitung A ein oder mehrere Verbindungen aus der Gruppe quartäre Salze und Alkylimidazoline eingesetzt werden.

10. Haarpflegeprodukt nach einem der Ansprüche 1 bis 9, **dadurch gekennzeichnet, dass** als Verbindungen aus der Gruppe Alkylamine, ethoxilierten Amine, quartären Salze und Alkylimidazoline der in der Zubereitung A quartäre Ammoniumsalze eingesetzt werden.

## Claims

1. Cosmetic hair care product comprising
a) a cosmetic hair care preparation A in an amount of from 75 to 25% by weight of the total preparation **characterized in that**, in the preparation A, one or more compounds from the group of the C₈-C₂₂ alkylhydroxy compounds in a total concentration of from 0.1 to 10% by weight, based on the total weight of preparation A, are used as fatty alcohols, and one or more compounds from the group of alkylamines, ethoxylated amines, quaternary salts and alkylimidazolines in a total concentration of from 0.1 to 8% by weight, based on the total weight of preparation A, are used as quaternary ammonium compounds,
b) a preparation B which comprises active ingredients for hair care and/or hair styling, in an amount of from 25 to 75% by weight of the total preparation, where, in preparation B, one or more compounds from the group of nonionic, cationic, anionic or amphoteric polymers are used as active ingredients for hair care and/or hair styling in a total concentration of from 0.01 to 10% by weight, based on the total weight of preparation B, where
i) the two preparations A and B are stored in separate chambers of a common packaging container and are used from this,
ii) both preparations are removed from the packaging container either through a common opening at the same time and both preparations are either mixed prior to exiting from the removal opening or emerge from the removal opening in the form of a striped overall preparation, or are removed from two separate openings.

2. Hair care product according to Claim 1, **characterized in that** a hair treatment is used as preparation A.

3. Hair care product according to one of Claims 1 or 2, **characterized in that** a hair setting composition is used as preparation B.

4. Hair care product according to one of Claims 1 to 3, **characterized in that** a gel-like hair setting composition is used as preparation B.

5. Hair care product according to one of Claims 1 to 4, **characterized in that**, in the preparation A, one or more compounds from the group of completely neutralized, partially neutralized or unneutralized, branched and/or unbranched, saturated and/or unsaturated fatty acids having a chain length of from 10 to 40 carbon atoms, polyethoxylated fatty acid esters, saturated and/or unsaturated, branched and/or unbranched fatty alcohols having a chain length of from 10 to 40 carbon atoms in a total concentration of from 0.1 to 10% by weight, based on the total weight of preparation A, are used as emulsifiers.

6. Hair care product according to one of Claims 1 to 5, **characterized in that**, in preparation B, film formers are used as active ingredients.

7. Hair care product according to one of Claims 1 to 6, **characterized in that** one or more compounds from the group of nonionic and amphoteric polymers as active ingredients for hair care and/or hair styling are used as polymers in preparation B.

8. Hair care product according to one of Claims 1 to 7, **characterized in that** one or more compounds from the group consisting of PVP/VA copolymer, PVP/dimethylaminoethyl methacrylate copolymer are used as polymers in preparation B.

9. Hair care product according to one of Claims 1 to 8, **characterized in that** one or more compounds from the group consisting of quaternary salts and alkylimidazolines are used as compounds from the group consisting of alkylamines, ethoxylated amines, quaternary salts and alkylimidazolines in preparation A.

10. Hair care product according to one of Claims 1 to 9, **characterized in that** quaternary ammonium salts are used as compounds from the group consisting of alkylamines, ethoxylated amines, quaternary salts and alkylimidazolines in preparation A.

## Revendications

1. Produit cosmétique de soin capillaire, contenant
a) une préparation cométique de soin capillaire A en une quantité de 75 à 25 % en poids de la préparation totale, **caractérisé en ce que** dans la préparation A on utilise en tant qu'alcools gras un ou plusieurs composés choisis dans le groupe des composés alkyl(C₈-C₂₂)hydroxy à une concentration totale de 0,1 à 10 % en poids, par rapport au poids total de la préparation A, et on utilise en tant que composés ammonium quaternaire un ou plusieurs composés choisis dans le groupe des alkylamines, amines éthoxylées, sels quaternaires et alkylimidazolines, à une concentration totale en 0,1 à 8 % en poids, par rapport au poids total de la préparation A,
b) une préparation B qui contient des actifs destinés au soin des cheveux et/ou à l'élaboration de la coiffure, en une quantité de 25 à 75 % en poids de la préparation totale, dans la préparation B un ou plusieurs composés, choisis dans le groupe des polymères non ioniques, cationiques, anioniques ou amphotères, étant utilisés en tant qu'actifs destinés au soin des cheveux et/ou à l'élaboration de la coiffure, à une concentration totale de 0,01 à 10 % en poids, par rapport au poids total de la préparation B,
i) les deux préparations A et B étant conservées dans des compartiments distincts d'un contenant d'emballage commun et étant utilisées en étant extraites de celui-ci,
ii) les deux préparations soit étant prélevées simultanément du contenant d'emballage par un orifice commun et les deux préparations soit étant mélangées avant la sortie de l'orifice de prélèvement, soit sortant sous forme d'une préparation totale en forme de bande de l'orifice de prélèvement, soit étant prélevées de deux orifices distincts.

2. Produit de soin capillaire selon la revendication 1, **caractérisé en ce qu'**une composition de traitement capillaire est utilisée en tant que préparation A.

3. Produit de soin capillaire selon la revendication 1 ou 2, **caractérisé en ce qu'**un fixateur pour cheveux est utilisé en tant que préparation B.

4. Produit de soin capillaire selon l'une quelconque des revendications 1 à 3, **caractérisé en ce que** la préparation B est un fixateur pour cheveux sous forme de gel.

5. Produit de soin capillaire selon l'une quelconque des revendications 1 à 4, **caractérisé en ce qu'**on utilise comme émulsifiants dans la préparation A un ou plusieurs composés choisis dans le groupe des acides gras ramifiés et/ou non ramifiés, saturés et/ou insaturés, totalement, partiellement on non neutralisés, ayant une longueur de chaîne de 10 à 40 atomes de carbone, des esters d'acides gras polyéthoxylés, des alcools gras ramifiés et/ou non ramifiés, saturés et/ou insaturés, ayant une longueur de chaîne de 10 à 40 atomes de carbone, à une concentration totale de 0,1 à 10 % en poids, par rapport au poids total de la préparation A.

6. Produit de soin capillaire selon l'une quelconque des revendications 1 à 5, **caractérisé en ce qu'**on utilise comme actifs dans la préparation B des agents filmogènes.

7. Produit de soin capillaire selon l'une quelconque des revendications 1 à 6, **caractérisé en ce qu'**on utilise comme actifs destinés au soin des cheveux et/ou à l'élaboration de la coiffure, en tant que polymères dans la préparation B, un ou plusieurs composés choisis dans le groupe des polymères non ioniques et des polymères amphotères.

8. Produit de soin capillaire selon l'une quelconque des revendications 1 à 7, **caractérisé en ce qu'**on utilise en tant que polymères dans la préparation B un ou plusieurs composés choisis dans le groupe constitué par un copolymère PVP/VA et un copolymère PVP/méthacrylate de diméthylaminoéthyle.

9. Produit de soin capillaire selon l'une quelconque des revendications 1 à 8, **caractérisé en ce qu'**on utilise en tant que composés choisis dans le groupe des alkylamines, amines éthoxylées, sels quaternaires et alkylimidazolines, dans la préparation A, un ou plusieurs composé choisis dans le groupe des sels quaternaires et des alkylimidazolines.

10. Produit de soin capillaire selon l'une quelconque des revendications 1 à 9, **caractérisé en ce qu'**on utilise des sels d'ammonium quaternaires en tant que composés choisis dans le groupe des alkylamines, amines éthoxylées, sels quaternaires et alkylimidazolines, dans la préparation A.
